(19) Europäisches Patentamt
European Patent Office
Office européen des brevets

(11) Veröffentlichungsnummer : **0 029 413**
**B1**

# (12) EUROPÄISCHE PATENTSCHRIFT

(45) Veröffentlichungstag der Patentschrift : **14.09.83**

(21) Anmeldenummer : **80810351.9**

(22) Anmeldetag : **14.11.80**

(51) Int. Cl.³ : **C 09 B 57/04**, C 07 D209/50, C 08 K 5/00

## (54) Iminoisoindolinonpigmente, Verfahren zu deren Herstellung und deren Verwendung.

(30) Priorität : **20.11.79 CH 10343/79**

(43) Veröffentlichungstag der Anmeldung : **27.05.81 Patentblatt 81/21**

(45) Bekanntmachung des Hinweises auf die Patenterteilung : **14.09.83 Patentblatt 83/37**

(84) Benannte Vertragsstaaten : **CH DE FR GB IT LI**

(56) Entgegenhaltungen :
**DE A 2 518 892**
**FR A 2 274 662**
**PL A 67 743**

**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Patentabteilung Postfach**
**CH-4002 Basel (CH)**

(72) Erfinder : **Model, Ernst, Dr.**
**Blotzheimerstrasse 69**
**CH-4055 Basel (CH)**

EP 0 029 413 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Iminoisoindolinonpigmente, Verfahren zu deren Herstellung und deren Verwendung

Gegenstand der vorliegenden Erfindung sind neue wertvolle Iminoisoindolinonpigmente der Formel

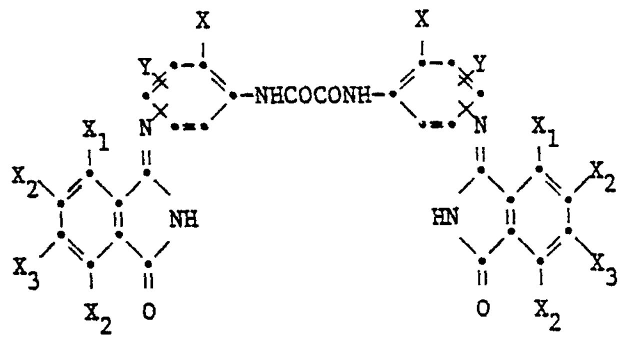

(1)

worin X und Y Wasserstoff, Halogen, Alkyl oder Alkoxy mit 1 bis 4 C-Atomen, die $X_2$ Chlor oder Brom und $X_1$ und $X_3$ Chlor, Brom, Alkoxy mit 1 bis 4 C-Atomen oder Aryloxy bedeuten. Von besonderem Interesse sind Iminoisoindolinonpigmente der Formel

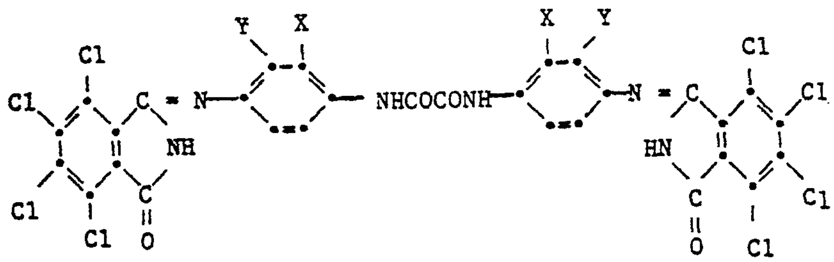

(2)

worin X Wasserstoff oder Methoxy und vorzugsweise Chlor oder Methyl und Y Wasserstoff, Methyl oder Chlor bedeuten.

Ausserdem von Interesse ist das Iminoisoindolinonpigment der Formel

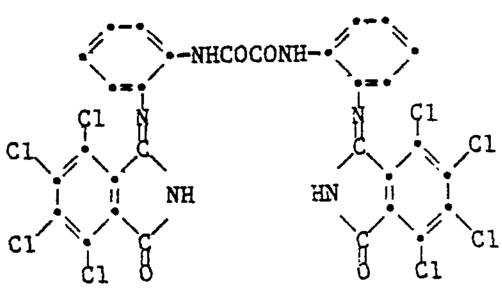

(3)

Die Iminoisoindolinonpigmente der Formel 1 werden dadurch hergestellt, dass man ein Isoindolinon der Formel

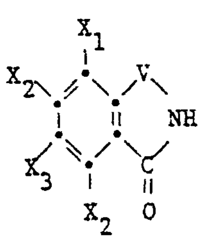

(4)

worin V eine Gruppe der Formel

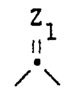 oder 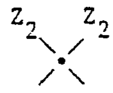 (5)

(a) (b)

bedeutet, in der $Z_1$ für eine Imino- der Thiogruppe und die $Z_2$ für Halogen, Alkoxy- oder sekundäre Aminogruppen stehen, mit einem Diamin der Formel

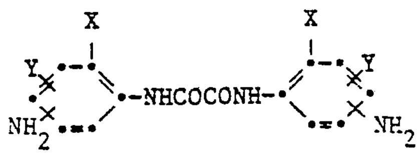

(6)

worin X und Y Wasserstoff, Halogen, Alkyl oder Alkoxy mit 1 bis 4 C-Atomen bedeuten, im Molverhältnis 2 : 1 kondensiert.

Als Ausgangsstoffe verwendet man vorzugsweise Isoindolinone der Formel

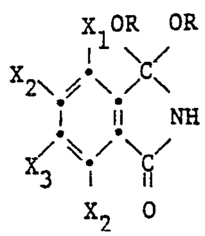

(7)

worin R Alkyl mit 1 bis 4 C-Atomen bedeutet, und $X_1$ bis $X_3$ die angegebene Bedeutung haben. Insbesondere geht man von Isoindolinonen der Formel 7 aus, worin $X_1$ bis $X_3$ Chlor oder Brom bedeuten.

Als Beispiele für Ausgangsstoffe der Formel 7 seien genannt :

3-Imino-4,5,6,7-tetrachlor-isoindolinon,
3-Imino-5,7-dichlor-4,6-dimethoxy-isoindolinon,
3,3,4,5,6,7-Hexachlor-isoindolinon,
3,3-Dimethoxy-5,7-dichlor-4,6-dimethoxy-isoindolinon,
3,3-Dimethoxy-4,5,7-trichlor-6-methoxy-isoindolinon,
3,3-Dimethoxy-4,5,6,7-tetrachlor-isoindolinon,
3,3-Dimethoxy-4,5,6,7-tetrabrom-isoindolinon,
3,3-Dimethoxy-4,5,7-trichlor-6-äthoxy-isoindolinon,
3,3-Dimethoxy-4,5,7-trichlor-6-n-propoxy-isoindolinon,
3,3-Dimethoxy-4,5,7-trichlor-6-n-butoxy-isoindolinon,
3,3-Dimethoxy-4,5,7-trichlor-6-phenoxy-isoindolinon.

Die genannten Isoindolinone stellen bekannte Verbindungen dar. Die Kondensation des Isoindolinons mit dem Diamin erfolgt teilweise in der Kälte, gegebenenfalls unter Erwärmen der innig vermischten Komponenten, vorzugsweise in Gegenwart inerter organischer Lösungsmittel.

Geht man von 3-Imino-, 3-Thio- oder 3,3-Bis-sek.amino-4,5,6,7-tetrachlorisoindolin-1-onen oder von Alkalisalzen der 3,3-Dialkoxy-4,5,6,7-tetrahalogenisoindolin-1-onen aus, so verwendet man vorteilhaft mit Wasser mischbare organische Lösungsmittel, z. B. niederaliphatische Alkohole, wie niedere Alkanole, beispielsweise Methanol, Isopropanol oder Butanol ; niedere cyclische Aether, wie Dioxan, Aethylenglykolmonomethyläther ; niedere aliphatische Ketone, wie Aceton ; Dimethylformamid oder Dimethylacetamid. Die Kondensation erfolgt hierbei schon bei verhältnismässig tiefen Temperaturen. Vorteilhaft arbeitet mann in Gegenwart basenbindender Mittel ; als solche sind Beispielsweise niedere Fettsäuren, die dann gleichzeitig als Lösungsmittel dienen, insbesondere Essigsäure, zu erwähnen.

Bei Verwendung von 3,3,4,5,6,7-Hexahalogenisoindolin-1-onen bevorzugt man hydroxylgruppenfreie organische Lösungsmittel, wie Kohlenwasserstoffe, z. B. aromatische, wie Benzol, Toluol, Xylol, Tetrahydronaphthalin oder Diphenyl oder cycloaliphatische, z. B. Cyclohexan, dann auch Halogenkohlenwasserstoffe, wie aliphatische, z. B. Tetrachlorkohlenstoff oder Tetrachloräthylen, oder aromatische, wie Chlorbenzol oder Di- und Trichlorbenzole, ferner aromatische Nitrokohlenwasserstoffe, wie Nitrobenzole, Aether, und zwar aliphatische wie Dibutyläther, aromatische wie Diphenyläther, oder cyclische Aether wie Dioxan, ferner Ketone, wie Aceton, oder Ester, namentlich Ester niederer Fettsäuren mit niederen Alkanolen, wie Essigsäureäthylester in Gegenwart von säurebindenden Mitteln.

Als Diamine der Formel 6 verwendet man vorzugsweise

4,4'-Diamino-oxalsäure-dianilid
4,4'-Diamino-2,2'-dimethyl-oxalsäure-dianilid
4,4'-Diamino-2,2'-dichlor-oxalsäure-dianilid
4,4'-Diamino-2,2'-dimethoxy-oxalsäure-dianilid
4,4'-Diamino-2,2'-diäthoxy-oxalsäure-dianilid
2,2'-Diamino-oxalsäure-dianilid.

Die erwähnten Amine sind bekannt oder können nach bekannten Methoden erhalten werden, beispielsweise durch Umsetzung von Oxalylchlorid mit einem gegebenenfalls substituierten Nitroanilin und anschliessender Reduktion.

Die neuen Pigmente fallen unmittelbar nach ihrer Bildung aus dem Reaktionsmedium aus. Sie sind für gewisse Zwecke als Rohpigmente direkt verwendbar. Sie können aber auch nach an sich bekannten Methoden, beispielsweise durch Extrahieren mit organischen Lösungsmitteln oder durch Mahlen mit nachher wieder entfernbaren Mahlhilfsmitteln, z. B. Salzen oder durch alkalische Umfällung, in ihren Eigenschaften, besonders bezüglich Reinheit, Form und Deckkraft, noch verbessert werden.

Die neuen Iminoisoindolinon-Verbindungen stellen wertvolle Pigmente dar, die sich überraschenderweise trotz unterbrochener Konjugation in ihrer Strukturformel durch Glanz, eine hohe Farbstärke, hohe Farbtonreinheit und eine gute Verteilbarkeit auszeichnen. Ausserdem weisen sie gute Licht-, Wetter-, Ueberlackier-, Hitze- und Migrationsechteiten auf. Sie können in feinverteilter Form zum Pigmentieren von hochmolekularem organischem Material verwendet werden, z. B. in Celluloseäthern und -estern, wie Aethylcellulose, Acetylcellulose, Nitrocellulose, Polyamiden bzw. Polyurethanen, oder Polyestern, natürlichen Harzen oder Kunstharzen, z. B. Aminoplasten, insbesondere Harnstoff- und Melamin-Formaldehydharzen, Alkydharzen, Phenoplasten, Polycarbonaten, Polyolefinen, wie Polystyrol, Polyvinylchlorid, Polyäthylen, Polypropylen, Polyacrylnitril, Polyacrylsäureester, thermoplastische oder härtbare Acrylharze, Gummi, Casein, Silikon und Silikonharzen, einzeln oder in Mischungen. Dabei spielt es keine Rolle, ob die erwähnten hochmolekularen Verbindungen als plastische Massen, Schmelzen oder in Form von Spinnlösungen, Lacken oder Drucksfarben vorliegen. Je nach Verwendungszweck erweist es sich als vorteilhaft, die neuen Pigmente als Toner oder in Form von Präparaten zu verwenden.

Die bekannten isomeren Pigmente der DE-A-2 518 892 zeichnen sich durch die —CONHNHCO— Gruppe als Brückengleid aus.

## Beispiel 1

17,25 g 3,4,5,6-Tetrachlor-1-cyanbenzoesäuremethylester werden mit 57,5 ml einer 1n-Natriummethylatlösung in Methanol zu einer klaren Lösung verrührt. Es entsteht das Natriumsalz des 3,3-Dimethoxy-4,5,6,7-tetrachlorisoindolin-1-ons. Unter gutem Rühren werden nun 7,5 g Oxalyl-bis-(4-amino-2-methylanilid) — fein gemahlen und gesiebt —, 100 ml o-Dichlorbenzol und 25 ml Dimethylformamid eingetragen. Bei einer Badtemperatur von 130 °C wird die Innentemperatur unter Abdestillieren von Methanol innert ca. 30 Minuten auf 100 °C erhöht, wobei sich, ohne dass eine klare Lösung entsteht, das Natriumsalz des Pigments abscheidet. Es wird mit weiteren 100 ml o-Dichlorbenzol verdünnt und nach einer Stunde Rühren bei 100 °C mit 20 ml Eisessig angesäuert. Nun wird die Temperatur auf 140-150 °C erhöht und während 2 Stunden gehalten. Das unlösliche Pigment wird bei 120 °C abfiltriert, mit heissem Methanol, Aceton und heissem Wasser gewaschen und getrocknet. Man erhält 20,2 g eines farbstarken Gelbpigments, das in dieser Form zur Färbung von Kunststoffen und zur Herstellung von Druckpasten und Farblacken verwendet werden kann. Die erhaltenen Gelbfärbungen zeichnen sich durch hervorragende Echtheiten aus.

## Beispiel 2

Verwendet man in Beispiel 1 anstelle von 3,4,5,6-Tetrachlor- 3,4,5,6-Tetrabrom-o-cyanbenzoesäuremethylester, dann erhält man ein Gelbpigment mit ähnlichen Eigenschaften.

## Beispiel 3

Verwendet man in Beispiel 1 anstelle des 3,4,5,6-Tetrachlor-o-cyanbenzoesäuremethylester äquimolare Mengen 3,4,6-Trichlor-5-methoxy-o-cyanbenzoesäuremethylester, dann erhält man ein Gelbpigment, das in Kunststoffe, Druckpasten und Farblacke eingearbeitet, ähnlich gute Eigenschaften aufweist.

Beispiel 4-21

In der folgenden Tabelle sind weitere Pigmente aufgeführt, die man durch Kondensation von 3,3-Dimethoxy-4,5,6,7-tetrachlorisoindolin-1-on mit einem Diamin der Formel

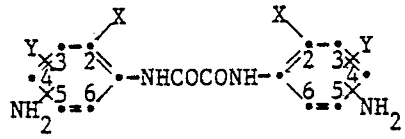

erhält, wobei die $NH_2$ und Y die in der Tabelle angegebene Stellung einnehmen und X und Y die angegebene Bedeutung haben.

| Nr. | $NH_2$ | X | Y | Nuance |
|---|---|---|---|---|
| 4 | 2,2' | H | H | gelb |
| 5 | 2,2' | H | 4,4' - Cl | grünstichiges gelb |
| 6 | 2,2' | H | 4,4' - $CH_3$ | gelb |
| 7 | 3,3' | H | H | grünstichiges gelb |
| 8 | 3,3' | H | 4,4' - $CH_3$ | grünstichiges gelb |
| 9 | 5,5' | $CH_3$ | H | grünstichiges gelb |
| 10 | 4,4' | H | H | gelb |
| 11 | 4,4' | $OCH_3$ | H | gelb |
| 12 | 4,4' | H | 3,3' - $CH_3$ | gelb |
| 13 | 4,4' | Cl | H | grünstichiges gelb |
| 14 | 4,4' | $CH_3$ | 5,5' - $CH_3$ | gelb |
| 15 | 4,4' | $CH_3$ | 3,3' - $CH_3$ | gelb |
| 16 | 4,4' | $OCH_3$ | 5,5' - $CH_3$ | gelb |
| 17 | 4,4' | $CH_3$ | 3,3' - Cl | gelb |
| 18 | 4,4' | $OCH_3$ | 5,5' - $OCH_3$ | orange |
| 19 | 4,4' | $OC_2H_5$ | 5,5' - $OC_2H_5$ | orange |
| 20 | 4,4' | Cl | 3,3' - Cl | grünstichiges gelb |
| 21 | 4,4' | Cl | 5,5' - Cl | grünstichiges gelb |

Beispiel 22

2 g des nach Beispiel 10 hergestellen Pigments werden mit 36 g Tonerdedehydrat, 60 g Leinölfirnis von mittlerer Viskosität und 2 g Kobaltlinoleat auf dem Dreiwalzenstuhl angerieben. Die mit der entstehenden Farbpaste erzeugten gelben Drucke sind farbstark und hervorragend lichtecht.

Beispiel 23

0,6 g des nach Beispiel 13 hergestellten Pigments werden mit 67 g Polyvinylchlorid, 33 g Dioctylphthalat, 2 g Dibutylzinndilaurat und 2 g Titandioxyd zusammengemischt und auf einem Walzenstuhl während 15 Minuten bei 160 °C zu einer dünnen Folie verarbeitet. Die so erzeugte Gelbfärbung ist farbstark, migrations-, hitze- und lichtecht.

Beispiel 24

10 g Titandioxyd und 2 g des nach Beispiel 1 hergestellten Pigments werden mit 88 g einer Mischung von 26,4 g Kokosalkydharz, 24,0 g Melamin-Formaldehydharz (50 % Festkörpergehalt), 8,8 g Aethylenglykolmonomethyläther und 28,8 g Xylol während 48 Stunden in einer Kugelmühle vermahlen.

Wird dieser Lack auf eine Aluminiumfolie gespritzt, 30 Minuten bei Raumtemperatur vorgestrocknet und dann während 30 Minuten bei 120 °C eingebrannt, dann erhält man eine Gelblackierung, die sich bei grosser Farbstärke durch eine sehr gute Ueberlackier-, Licht- und Wetterechtheit auszeichnet.

**Ansprüche**

1. Iminoisoindolinopigmente der Formel

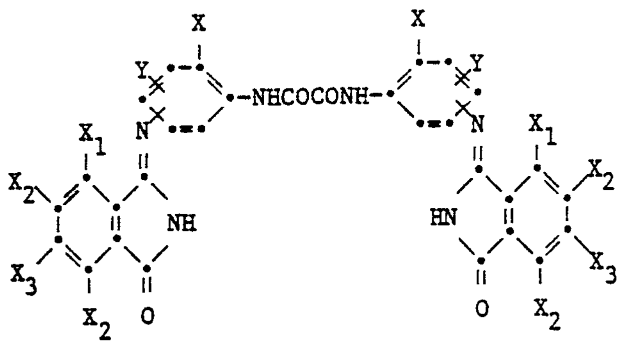

(1)

worin X und Y Wasserstoff, Halogen, Alkyl oder Alkoxy mit 1 bis 4 C-Atomen, die $X_2$ Chlor oder Brom und $X_1$ und $X_3$ Chlor, Brom, Alkoxy mit 1 bis 4 C-Atomen oder Aryloxy bedeuten.

2. Iminoisoindolinonpigmente gemäss Anspruch 1, gekennzeichnet durch die Formel

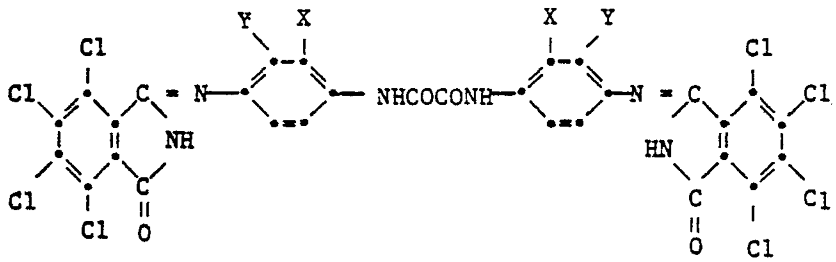

(2)

worin X Wasserstoff, Chlor, Methyl oder Methoxy und Y Wasserstoff, Methyl oder Chlor bedeuten.

3. Iminoisoindolinonpigmente gemäss Anspruch 2, dadurch gekennzeichnet, dass X Wasserstoff, Chlor oder Methyl und Y Wasserstoff bedeuten.

4. Iminoisoindolinonpigment gemäss Anspruch 1, gekennzeichnet durch die Formel

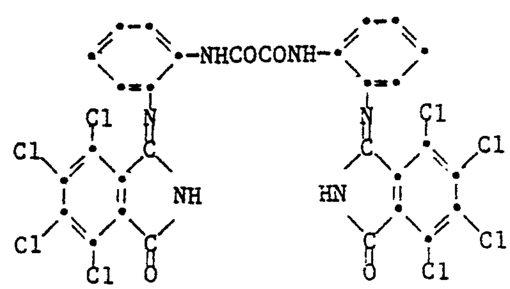

5. Verfahren zur Herstellung der Iminoisoindolinopigmente gemäss Anspruch 1, dadurch gekennzeichnet, dass man ein Isoindolinon der Formel

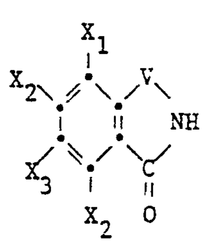

worin V eine Gruppe der Formel

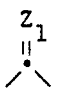

oder

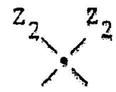

bedeutet, in der $Z_1$ für eine Imino- oder Thiogruppe und die $Z_2$ für Halogen, Alkoxy- oder sekundäre Aminogruppen stehen, mit einem Diamin der Formel

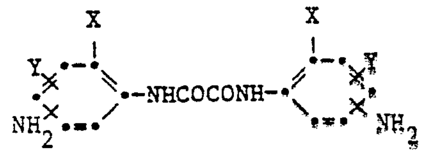

worin X und Y Wasserstoff, Halogen, Alkyl oder Alkoxy mit 1 bis 4 C-Atomen bedeuten, im Molverhältnis 2 : 1 kondensiert.

6. Verfahren gemäss Anspruch 5, dadurch gekennzeichnet, dass man von einem Isoindolinon ausgeht, worin die Reste $X_1$, $X_2$ und $X_3$ Chlor bedeuten.

7. Verfahren gemäss Anspruch 5, dadurch gekennzeichnet, dass man von einem Diamin der Formel

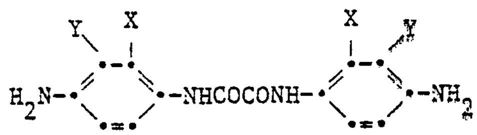

ausgeht, worin X Wasserstoff, Chlor, Methyl oder Methoxy und Y Wasserstoff, Methyl oder Chlor bedeuten.

8. Verfahren gemäss Anspruch 5, dadurch gekennzeichnet, dass man von einem Diamin der Formel

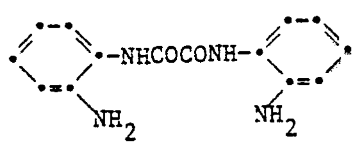

ausgeht.

9. Verwendung der Iminoisoindolinonpigmente gemäss Anspruch 1 zum Pigmentieren von hochmolekularem organischem Material ausserhalb der Textilindustrie.

10. Hochmolekulares organisches Material, enthaltend ein Iminoisoindolinonpigment gemäss Anspruch 1.

**Claims**

1. An iminoisoindolinone pigment of the formula

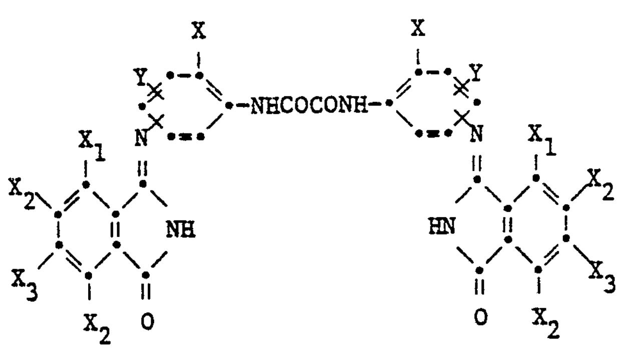

(1)

wherein X and Y are halogen, alkyl or alkoxy, each of 1 to 4 carbon atoms, $X_2$ is chlorine or bromine, and $X_1$ and $X_3$ are chlorine, bromine, alkoxy of 1 to 4 carbon atoms, or aryloxy.

2. An iminoisoindolinone pigment according to claim 1 of the formula

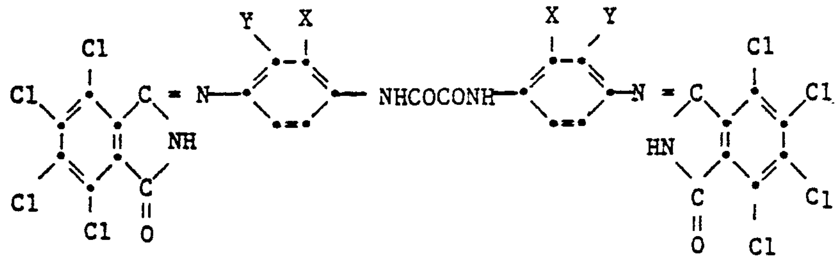

(2)

wherein X is hydrogen, chlorine, methyl or methoxy, and Y is hydrogen, methyl or chlorine.

3. An iminoisoindolinone pigment according to claim 2, wherein X is hydrogen, chlorine or methyl, and Y is hydrogen.

4. An iminoisoindolinone pigment according to claim 1 of the formula

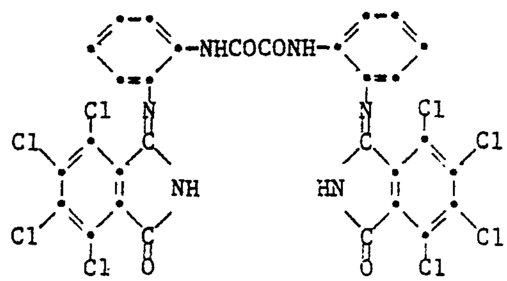

5. A process for the production of an iminoisoindolinone pigment according to claim 1, which process comprises condensing an isoindolinone of the formula

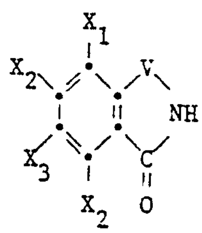

in which V is a group of the formula

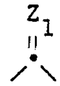

or

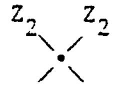

wherein $Z_1$ is an imino or thio group and $Z_2$ is halogen, alkoxy or a secondary amino group, with a diamine of the formula

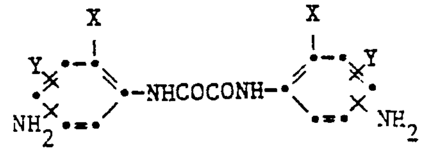

wherein X and Y are hydrogen, halogen, alkyl or alkoxy, each of 1 to 4 carbon atoms, in the molar ratio 2 : 1.

6. A process according to claim 5, wherein the starting material is an isoindolinone in which $X_1$, $X_2$ and $X_3$ are chlorine.

7. A process according to claim 5, wherein the starting material is a diamine of the formula

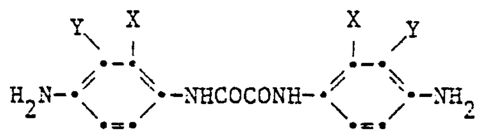

wherein X is hydrogen, chlorine, methyl or methoxy, and Y is hydrogen, methyl or chlorine.

8. A process according to claim 5, wherein the starting material is a diamine of the formula

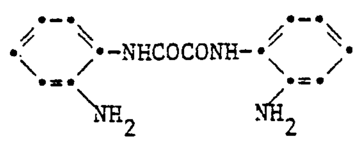

9. A method of pigmenting organic material of high molecular weight in sectors outside the textile industry, which method comprises the use of an iminoisoindolinone pigment according to claim 1.

10. Organic material of high molecular weight containing an iminoisoindolinone pigment according to claim 1.

**Revendications**

1. Pigments dérivant de l'imino-iso-indolinone qui répondent à la formule

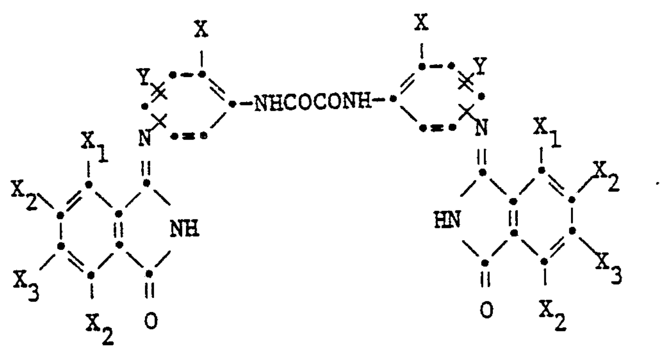

(1)

dans laquelle X et Y représentent chacun un atome d'hydrogène ou d'halogène ou un radical alkyle ou alcoxy contenant de 1 à 4 atomes de carbone, les $X_2$ représentent chacun le chlore ou le brome, et $X_1$ et $X_3$ représentent chacun le chlore, le brome, un alcoxy en $C_1$-$C_4$ ou un aryloxy.

2. Pigments imino-iso-indolinoniques selon la revendication 1, caractérisés en ce qu'ils répondent à la formule

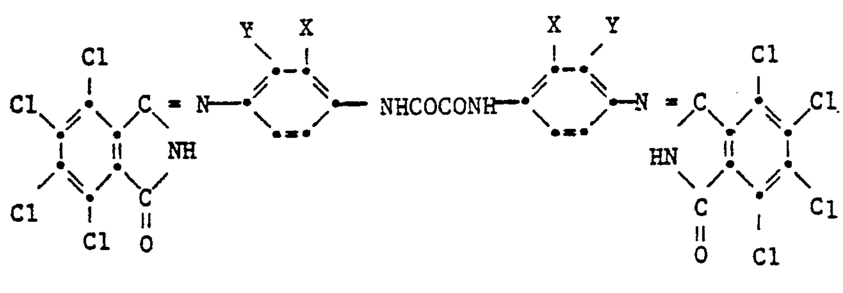

(2)

dans laquelle X représente l'hydrogène, le chlore, un méthyle ou un méthoxy et Y représénte d'hydrogène, un méthyle ou le chlore.

3. Pigments imino-iso-indolinoniques selon la revendication 2, caractérisés en ce que X représente l'hydrogène, le chlore ou un méthyle et Y représente l'hydrogène.

4. Pigment imino-iso-indolinonique selon la revendication 1, en l'espèce celui qui répond à la formule suivante

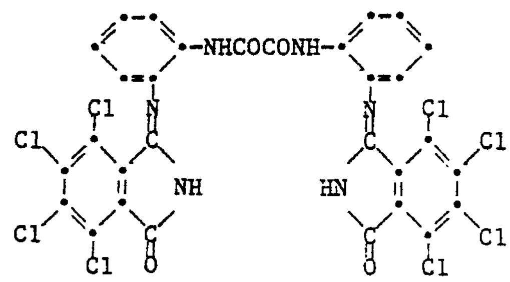

5. Procédé de préparation des pigments imino-iso-indolinoniques selon la revendication 1, caractérisé en ce qu'on condense une iso-indolinone répondant à la formule

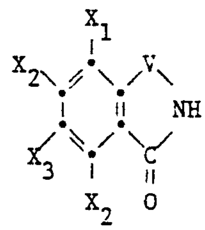

dans laquelle V représente un radical répondant à l'une des formules

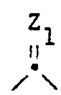 et 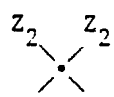

dans lesquelles $Z_1$ représente un groupe imino ou thio et les $Z_2$ représentent chacun un halogène, un radical alcoxy ou un radical amino secondaire, avec une diamine répondant à la formule

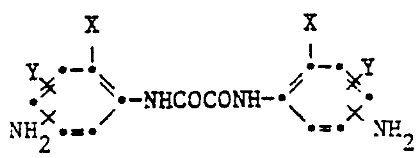

dans laquelle X et Y représentent chacun un atome d'hydrogène ou d'halogène ou un radical alkyl ou alcoxy contenant de 1 à 4 atomes de carbone, dans un rapport molaire égal à 2 : 1.

6. Procédé selon la revendication 5, caractérisé en ce qu'on part d'une iso-indolinone dans laquelle $X_1$, $X_2$ et $X_3$ représentent chacun le chlore.

7. Procédé selon la revendication 5, caractérisé en ce qu'on part d'une diamine répondant à la formule

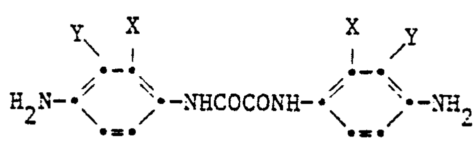

dans laquelle X représente l'hydrogène, le chlore, un méthyle ou un méthoxy et Y représente l'hydrogène, un méthyle ou le chlore.

8. Procédé selon la revendication 5, caractérisé en ce qu'on part de la diamine qui répond à la formule suivante

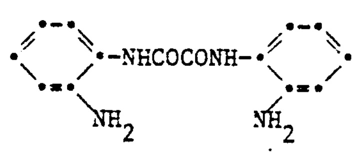

9. Application des pigments imino-iso-indolinoniques selon la revendication 1 pour la pigmentation de matières organiques macro-moléculaires en dehors de l'industrie textile.

10. Matière organique macro-moléculaire qui contient un pigment imino-iso-indolinonique selon la revendication 1.